# EUROPEAN PATENT SPECIFICATION

(11) **EP 4 201 480 B1**
(45) Date of publication and mention of the grant of the patent: **22.11.2023**
(21) Application number: 21217223.3
(22) Date of filing: 23.12.2021
(51) Int. Cl.: A61N 7/00, G02B 3/14, G02B 26/00, A61H 39/00, A61N 5/06

(54) **ULTRASONIC WAVE ACUPUNCTURE DEVICE**
ULTRASCHALLWELLEN-AKUPUNKTURGERÄT
DISPOSITIF D'ACUPUNCTURE PAR ONDES ULTRASONIQUES

(43) Date of publication of application: 28.06.2023
(73) Proprietor: Industrial Technology Research Institute, 310401 Hsinchu (TW)
(72) Inventor: CHEN, Chun-Jung, 310 Hsinchu County (TW); LEE, Kuo-Chun, 106 Taipei City (TW); CHIEN, Chih-Wei, 542 Nantou County (TW)
(74) Representative: Becker Kurig & Partner Patentanwälte mbB

(56) References cited:
- WO-A1-2010/038162
- WO-A1-2014/144923
- WO-A2-2007/125500
- WO-A2-2009/007900
- WO-A2-2009/027920
- CN-A- 101 284 163
- CN-A- 102 415 942
- CN-A- 105 496 763

## Description

### BACKGROUND

### Technical Field

The present disclosure relates to an ultrasonic wave acupuncture device.

### Description of Related Art

Acupuncture is a traditional Chinese treatment. It is effective in treating illness like motion sickness by stimulating specific acupoints by acupuncture and moxibustion. Motion sickness is a disorder of the balance system. The system responsible for balance in the human body includes the eyes and inner ears. When taking a vehicle, the rotation or turning of the vehicle, the start of the car or the acceleration or deceleration brake, and the shaking or bumping of the ship may cause confusion in the balance mechanism of the vision and inner ears, bringing symptoms like nausea and vomiting, seasickness in cars, planes, and ships. In addition, the use of Virtual Reality (VR) or Augmented Reality (AR) may also cause symptoms such as dizziness.

Statistically speaking, about 25 to 30% of people in the world have suffered from motion sickness, and children aged 2 to 12 are the population most prone to motion sickness. Also, adult women are more likely to suffer from motion sickness than men, especially women who are on menstrual periods or pregnant. Patients with migraine, inner ear nerves incoordination, and central nervous system incoordination are all at high risk of motion sickness. Moreover, people with poor mental health, lack of sleep, or hangovers are also more likely to suffer from motion sickness.

The current treatments for motion sickness can be roughly divided into chemical treatments and physical treatments. Chemical treatments include taking drugs 30 to 60 minutes before taking a vehicle to reduce the sensitivity of the vestibule of the inner ear while making the central nervous system antiemetic. Still, the use of drugs may cause side effects. For example, motion sickness drugs as a blocker of parasympathetic nerves in the autonomic nerves reduces the transmission of acetylcholine, and after taking the motion sickness medicine, there may be side effects such as drowsiness, dry mouth, blurred vision, difficulty urinating, constipation, and possible heart palpitations. The physical treatments include the use of anti-faint patches 4 hours before the departure of the vehicle, or the use of traditional Chinese medicine acupoints, anti-faint bracelets, and other treatments. But generally speaking, motion sickness has not been effectively relieved.

Acupuncture and moxibustion in Chinese medicine are considered effective treatments for motion sickness, but acupuncture also has some disadvantages. For example, acupuncture may cause patients strong irritation and pain. As the skin is punctured when practicing acupuncture, it may cause infection easily. The technical difficulty of the needle technique of acupuncture also makes it difficult to replicate the effect of needling. In addition, the smoke of the moxibustion treatment and improper use can easily leave scars on the skin, and cause air pollution and permanent scars for the patient.
CN 105 496 763 A discloses a a two-dimensional ultrasonic phased array acupuncture system, comprising a phase control transmitting module, and a two-dimensional phased array transducer; the phase control transmitting module receives a focusing indicating signal which indicates change of a focusing direction and/or a focusing depth and a focusing position; the phase control transmitting module generates ultrasonic pulse transmitting time sequences of different ultrasonic wafers.
CN 101 284 163 A discloses a focused ultrasonic-wave point treatment device, comprising a host machine and a treatment gun. The treatment gun is connected with the host machine through a connecting cable; an ultrasonic transducer that can generate focused ultrasonic-waves is arranged inside the treatment gun; the said ultrasonic transducer is a non-axial symmetry ultrasonic transducer, and in the treatment gun there is included an acoustic-field adjusting unit of the non-axial symmetry acoustic-field that leads the ultrasonic transducer to rotate.
CN 102 415 942 A discloses a an ultrasonic acupuncture instrument, which comprises a host machine and an ultrasound treatment head, wherein the ultrasound treatment head comprises an ultrasonic transducer, the host machine comprises a control unit and a drive unit, the control unit comprises a signal processing module, a main frequency control module, and a needle insertion control module used for setting the needle insertion dosage of ultrasound in the ultrasonic transducer into a fixed value and transmitting the value to the signal processing module.
WO 2014/144923 A1 discloses a device for delivering light and/or ultrasound across a skin surface. The device has a layered structure comprising a light source and/or an ultrasonic transducer. The light source comprises an organic light emitting diode or a plurality of printed light emitting diodes and the ultrasonic transducer comprises a piezoelectric coating. The device is electrically coupled to a controller, which is electrically coupled to one or more sensors in contact with the skin surface. The controller is operable to receive sensor data from the sensor and dynamically control the device in response to the received sensor data.
WO 2009/027920 A2 discloses an ultrasound device for detecting presence or absence of cavitation events. An ultrasound transducer receives emitted and reflected ultrasound waves from tissues within a specific target volume and the received ultrasound waves are subsequently converted into a detection signal. A control unit then processes the detected signal for determining whether a cavitation event is detected. A liquid lens having an adjustable focal point is placed in front of the ultrasound transducer such that the received ultrasound waves go through the liquid lens prior to be received by the ultrasound transducer.
WO 2009/007900 A2 discloses an ultrasonic assembly suited for attachment to a catheter. The ultrasonic assembly includes an adjustable ultrasonic focus mechanism arranged in connection with the ultrasonic transducer to adjust focus of ultrasonic waves generated by the transducer. The ultrasonic focus mechanism includes a fluid focus lens with at least two fluids separated by an interface such that ultrasonic waves are substantially reflected at the interface. At least two electrodes are arranged in connection with the fluid focus lens so as to allow adjustment of the interface shape, when a voltage is applied to the electrodes.
WO 2010/038162 A1 discloses a system and method for treating tissue, using an ultrasound therapy system. An ultrasonic applicator has at least one transducer element. The steering and focusing of ultrasound beams uses at least one variable focus lens, which may be a fluid focus lens, attached to each transducer element so that focus of the variable focus lens is controlled by a voltage signal. A treatment controller receives input from an imaging means and controls the voltage applied to the variable focus lens. The treatment controller controls the lens voltage signals, at least partially determined by the input from the imaging means, and directs an ultrasonic treatment beam emitted by the transducer element.
WO 2007/125500 A2 discloses a acoustic probe including an acoustic transducer with a plurality of acoustic transducer elements arranged in a one-dimensional array; and a variably- refracting acoustic lens coupled to the acoustic transducer.

### SUMMARY

The present invention is provided by the appended claims. The following disclosure serves a better understanding of the present invention. The disclosure provides an ultrasonic wave acupuncture device that stimulates acupoints with ultrasonic pulses non-invasively by arbitrarily dynamically adjusting the focus depth of the liquid lens of the ultrasonic wave acupuncture device to achieve the effect similar to acupuncture in traditional Chinese medicine.

According to some embodiments of the disclosure, provided is an ultrasonic wave acupuncture device, including: an ultrasonic source, adapted to generate ultrasonic waves; a liquid lens, adapted to focus the ultrasonic waves; an ultrasonic-source controller, electrically connected to the ultrasonic source to generate a plurality of ultrasonic-source voltage pulses to control the frequency, amplitude, and pulse length of the ultrasonic waves generated by the ultrasonic source; a liquid-lens controller, electrically connected to the liquid lens to generate a plurality of liquid-lens voltage pulses to control the focal length of the liquid lens, so as to focus the ultrasound waves on a specific location; and a power supply, electrically connected to the ultrasonic-source controller and the liquid-lens controller, adapted to provide voltage to the ultrasonic-source controller to control the ultrasonic source, and provide voltage to the liquid-lens controller to control the liquid lens, wherein the ultrasonic-source voltage pulses generated by the ultrasonic-source controller and the liquid-lens voltage pulses generated by the liquid-lens controller are aligned with each other.

According to some embodiments of the disclosure, provided is an ultrasonic wave acupuncture device, including: an ultrasonic wave acupuncture device, including: an infrared source, adapted to generate far infrared rays; an ultrasonic source, adapted to generate ultrasonic waves, and the ultrasonic source having a hole to allow the far infrared rays to pass through; a liquid lens, adapted to focus the ultrasonic waves and the far infrared rays; an ultrasonic-source controller, electrically connected to the ultrasonic source to generate a plurality of ultrasonic-source voltage pulses, so as to control the frequency, amplitude, and pulse length of the ultrasonic waves generated by the ultrasonic source; an infrared-source controller, electrically connected to the infrared source to generate a plurality of infrared-source voltage pulses, so as to control the energy and pulse length of the far infrared rays generated by the infrared source; a liquid-lens controller, electrically connected to the liquid lens to generate a plurality of liquid-lens voltage pulses, so as to control the focal length of the liquid lens to focus the ultrasonic waves and the far infrared rays at a specific location; and a power supply, electrically connected to the infrared-source controller, the ultrasonic-source controller, and the liquid-lens controller, so as to provide voltage to the infrared-source controller to control the infrared source, to provide voltage to the ultrasonic-source controller to control the ultrasonic source, and to provide voltage to the liquid-lens controller to control the liquid lens, wherein when the ultrasonic source emits the ultrasonic wave, the infrared source turns off the far infrared rays, and when the infrared source emits the far infrared rays, the ultrasound source turns off the ultrasound waves, wherein the ultrasonic-source voltage pulses generated by the ultrasonic-source controller and the liquid-lens voltage pulses generated by the liquid-lens controller are aligned with each other, wherein the infrared-source voltage pulses of the infrared-source controller and the liquid-lens voltage pulses generated by the liquid-lens controller are aligned with each other.

Based on the above, the disclosure provides an ultrasonic wave acupuncture device. A liquid lens is adapted to focus the energy of the ultrasound pulse at one point, and the liquid lens has the characteristic of arbitrarily controlling the curvature of the liquid lens, so as to achieve the effect of different focus positions. The ultrasonic pulse has a needling effect similar to that in acupuncture by continuously changing the focal length of the liquid lens and generating a continuous energy focus point; moreover, it is a non-invasive process that does not need to puncture the skin. The ultrasonic wave acupuncture device provided by the disclosure also uses far infrared rays to stimulate specific acupoints through a liquid lens to achieve the effect similar to moxibustion (in which the mugwort is lit to heat and/or burn the acupoints, that is, the acupoints are stimulated by heating the acupoints). Therefore, this ultrasonic wave acupuncture device provides a soothing effect on some illness; for example, for those with motion sickness, it becomes unnecessary to take medicine and shoots to avoid sickness in a vehicle.

### BRIEF DESCRIPTION OF THE DRAWINGS

FIG. 1 is a schematic diagram of an ultrasonic wave acupuncture device according to some embodiments of the disclosure.
FIG. 2A to FIG. 2D are schematic diagrams of liquid lenses according to some embodiments of the disclosure.
FIG. 3 is a schematic diagram of a liquid lens according to some embodiments of the disclosure.
FIG. 4A and FIG. 4B are schematic diagrams of another liquid lens according to some embodiments of the disclosure.
FIG. 5 is a time sequence diagram of the voltage of the liquid lens and the voltage of the ultrasonic source according to some embodiments of the disclosure.
FIG. 6 is a time sequence diagram of the voltage of the liquid lens and the voltage of the ultrasonic source according to other embodiments of the disclosure.
FIG. 7 is a time sequence diagram of the voltage of the liquid lens and the voltage of the infrared source according to other embodiments of the disclosure.
FIG. 8 is an operation flowchart of the ultrasonic wave acupuncture device according to some embodiments of the disclosure.

### DESCRIPTION OF THE EMBODIMENTS

Please refer to the following embodiments and drawings for full comprehension of the disclosure. The disclosure can still be practiced in many different forms and should not be construed as limited to the embodiments described herein. In the drawings, for the sake of clarity, the components and their relative dimensions may not be drawn according to actual scale.

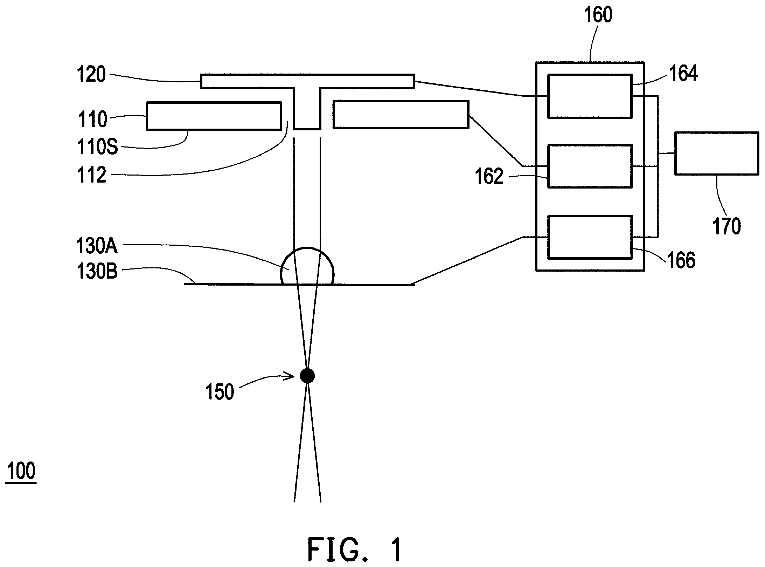

FIG. 1 is a schematic diagram of an ultrasonic wave acupuncture device according to some embodiments of the disclosure. As shown in FIG. 1, the ultrasonic wave acupuncture device 100 includes an ultrasonic source 110, a liquid lens 130A, and an electrode 130B. The ultrasonic wave acupuncture device 100 further includes a controller 160 and a power supply 170. The controller 160 includes an ultrasonic-source controller 162, an infrared-source controller 164, and a liquid-lens controller 166.

The ultrasonic source 110 is electrically connected to the ultrasonic-source controller 162. The ultrasonic source 110 generates ultrasonic waves by vibrating rapidly, and emits the ultrasonic waves in the form of pulses. The ultrasonic-source controller 162 generates a plurality of ultrasonic-source voltage pulses to control the frequency, amplitude, and pulse length of the ultrasonic waves generated by the ultrasonic source 110, and makes the ultrasonic source 110 emit ultrasonic waves in the form of pulses to produce acupuncture-like effects on specific acupoints. According to the selected mode, the ultrasonic-source controller 162 inputs a pulse voltage of a specific intensity to the ultrasonic source 110 such that the ultrasonic source 110 generates ultrasonic waves of a specific frequency and strength, and makes the ultrasonic source 110 emit ultrasonic waves in the form of pulses. According to some embodiments, the ultrasonic source 110 is a piezoelectric plate, and the material of the piezoelectric plate includes piezoelectric ceramic materials containing barium titanate (BaTiO₃), lead titanate (PbTiO₃) and lead zirconate titanate (Pb(ZrTi)O₃, PZT), or other materials with similar properties, but this disclosure is not limited thereto. According to some embodiments, the ultrasonic source 110 generates ultrasonic waves with a frequency of 1-3 MHz and a power range of 100-300 mW, but it is not limited thereto. According to some embodiments, the output power of the ultrasonic waves generated by the ultrasonic source 110 is preferably 230 mW. According to some embodiments, the pulse length of the ultrasonic pulse generated by the ultrasonic source 110 is in the range of 0.5 to 10 seconds, and the pulse interval is in the range of 1 to 30 seconds, but it is not limited thereto. According to some embodiments, the surface 110S of the ultrasonic source 110 facing the liquid lens 130A is a flat surface or a curved surface, but the disclosure is not limited thereto. When the surface 110S is a flat surface, the ultrasonic waves generated by the ultrasonic wave source 110 are focused by the liquid lens 130A to generate acupuncture-like stimulation to specific acupoints. When the surface 110S is a curved surface, it may be adapted to focus the ultrasonic waves generated by the ultrasonic source 110. According to some embodiments, the ultrasonic waves focused by the liquid lens 130A has a flare diameter of less than 0.5 mm after the ultrasonic waves are focused to generate acupuncture-like stimulation to specific acupoints.

The ultrasonic waves generated by the ultrasonic source 110 are focused on the focal point 150 through the liquid lens 130A. In this embodiment, the liquid lens 130A is a convex lens. Convex lenses are lenses with a thicker center and thinner edges, and they are divided into types like biconvex, plano-convex, and concave-convex. Convex lenses have the function of converging light (energy), so it is also called converging lenses. In this embodiment, the liquid lens 130A is a plano-convex lens. According to other embodiments, the liquid lens 130A may also be a biconvex lens, but the disclosure is not limited thereto.

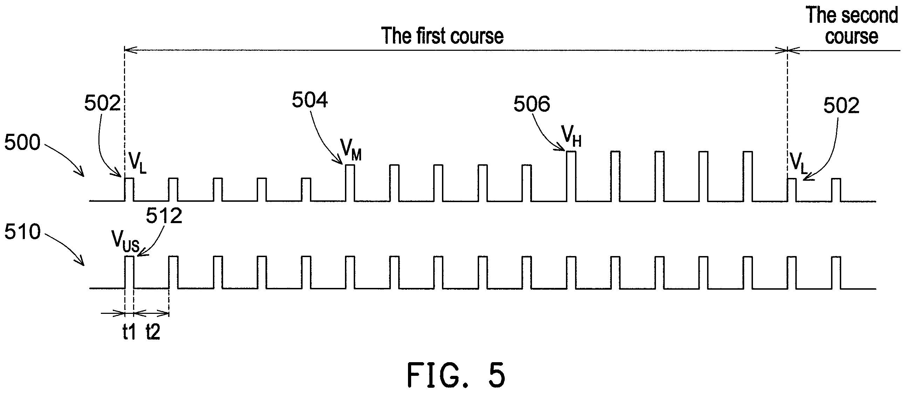

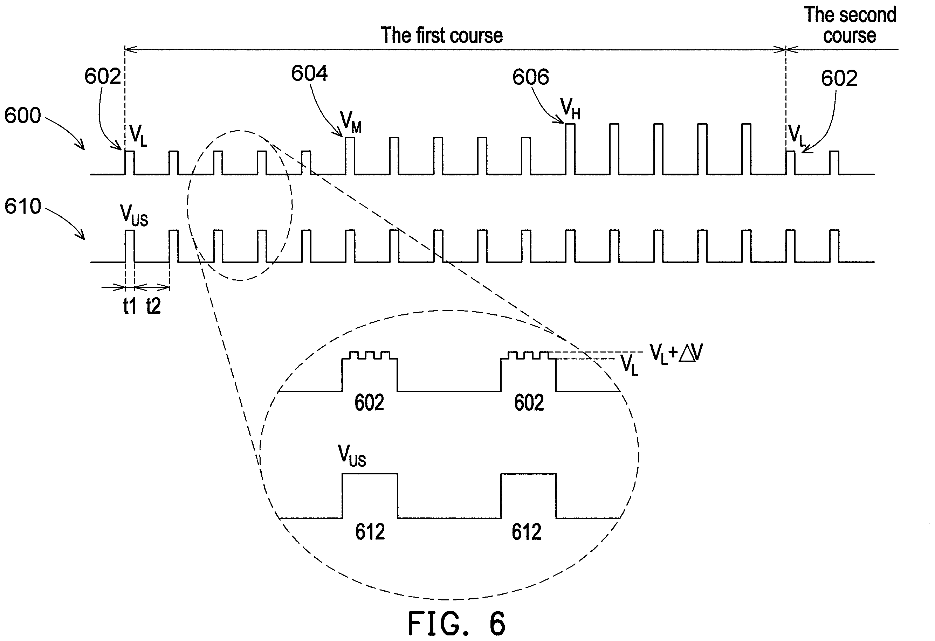

The liquid-lens controller 166 is electrically connected to the liquid lens 130A. The liquid-lens controller 166 generates a plurality of liquid-lens voltage pulses and inputs them to the liquid lens 130A through the electrode 130B to change the shape of the liquid lens 130A and to control the focal length of the liquid lens 130A, so that the ultrasonic waves are focused on a specific location to produce an effect similar to a needle insertion or a needle lifting. According to some embodiments, the ultrasonic-source voltage pulses generated by the ultrasonic-source controller 162 and the liquid-lens voltage pulses generated by the liquid-lens controller 166 are aligned with each other (which will be further described in FIG. 5 and FIG. 6). According to some embodiments, the liquid lens 130A is one or more liquid lenses, or other lenses that can change the focal length, but the disclosure is not limited thereto.

According to some embodiments, as shown in FIG. 1, the ultrasonic wave acupuncture device 100 further includes an infrared source 120 and an infrared-source controller 164. The infrared source 120 is electrically connected to the infrared-source controller 164. The infrared source 120 is located on the side of the ultrasonic source 110 facing away from the liquid lens 130A. In order to allow the far infrared rays generated by the infrared source 120 to penetrate the ultrasonic source 110, the ultrasonic source 110 is provided with a hole 112 to allow the far infrared rays emitted by the infrared source 120 to pass through. According to other embodiments, the infrared source 120 is located between the ultrasonic source 110 and the liquid lens 130A. When the infrared source 120 is located between the ultrasonic source 110 and the liquid lens 130A, the infrared source 120 shields part of the ultrasonic waves generated by the ultrasonic source 110, and thus the ultrasound source 110 needs to adjust the ultrasound emission power such that the specific acupoints receives sufficient ultrasound energy.

The infrared-source controller 164 generates a plurality of infrared-source voltage pulses to control the energy and the pulse length of the far infrared rays generated by the infrared source 120. The far infrared rays generated by the infrared source 120 are focused by the liquid lens 130A to heat specific acupoints and generate moxibustion-like stimulation. According to some embodiments, the infrared-source voltage pulses of the infrared-source controller 164 and the liquid-lens voltage pulses generated by the liquid-lens controller 166 are aligned with each other. According to some embodiments, the infrared source 120 is a light-emitting diode, or other components that generates far infrared rays, but the disclosure is not limited thereto. According to some embodiments, the wavelength range of the far infrared rays generated by the infrared source 120 is 8 to 14 µm, but it is not limited thereto.

The power supply 170 is electrically connected respectively to the ultrasonic-source controller 162, the infrared-source controller 164, and the liquid-lens controller 166 to provide control voltages to the ultrasonic-source controller 162, the infrared-source controller 164, and the liquid-lens controller 166 to control the ultrasonic source 110, the infrared source 120, and the liquid lens 130A, respectively.

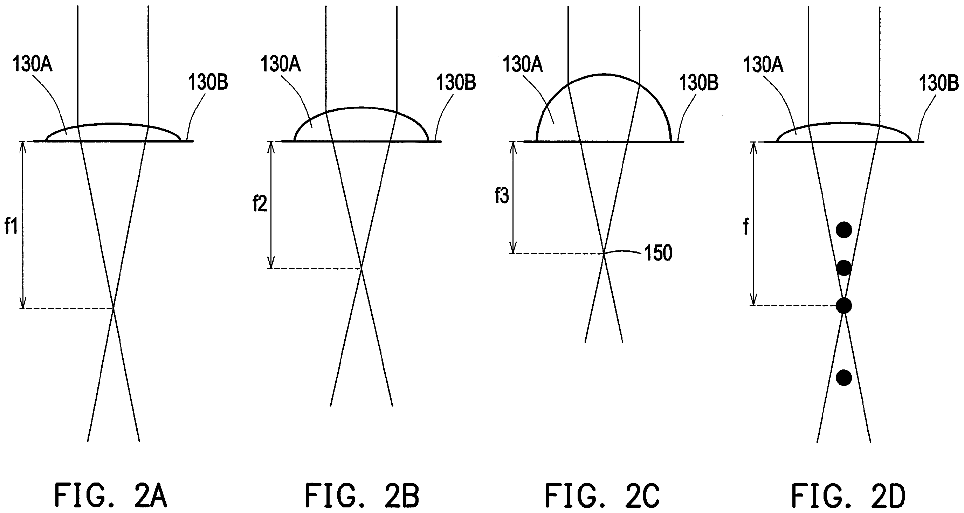

FIG. 2A to FIG. 2D are schematic diagrams of liquid lenses according to some embodiments of the disclosure. In FIG. 1, the liquid lens 130A changes the focal length of the lens by applying a voltage to the electrode 130B to form an electric field, so as to focus the ultrasonic waves generated by the ultrasonic source 110 or the far infrared rays generated by the infrared source 120. In FIG. 2A to FIG. 2C, as the electrode 130B provides different voltages, the liquid lens 130A produces different deformations, such that the liquid lens 130A has a different focal length. For example, in FIG. 2A, as the voltage of the electrode 130B passing through the liquid lens 130A is low, the liquid lens 130A has a small thickness at the moment, and the liquid lens 130A has a longer focal length f1 at this time. When the voltage of the electrode 130B gradually increases, the thickness of the liquid lens 130A also gradually increases, and the focal length of the liquid lens 130A gradually decreases, as shown in the focal length f2 in FIG. 2B and the focal length f3 in FIG. 2C. Therefore, as shown in FIG. 2D, by providing different voltages to the liquid lens 130A, the focal length f of the liquid lens 130A is changed. The focal length f changes along a straight line (the black dots in the figure are the focal points). The ultrasonic waves or the far infrared rays passing through the liquid lens 130A may be focused on a specific location and may be focused along a straight line.

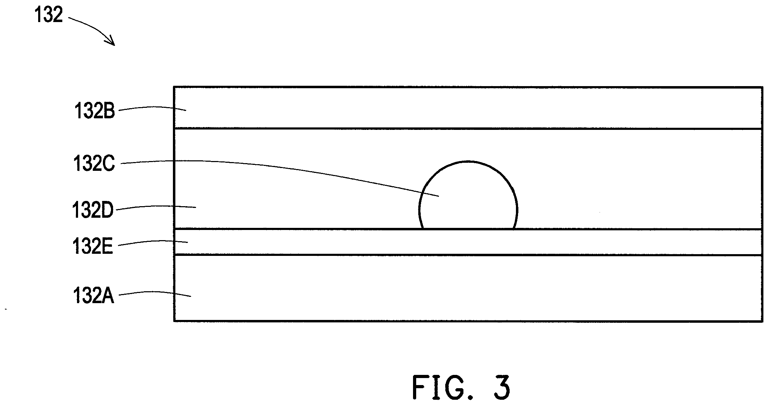

FIG. 3 is a schematic diagram of a liquid lens according to some embodiments of the disclosure. As shown in FIG. 3, the liquid lens 132 is an embodiment of the liquid lens 130A and the electrode 130B in FIG. 1. The liquid lens 132 includes an electrode 132A and an electrode 132B. Ultrasonic waves or far infrared rays enter the liquid lens 132 from the electrode 132B.

The liquid lens 132 further includes a lens liquid 132C and an encapsulating liquid 132D. The lens liquid 132C and the encapsulating liquid 132D are two immiscible liquids. According to some embodiments, the lens liquid 132C is oil, and the encapsulating liquid 132D is water. The lens liquid 132C and the encapsulating liquid 132D are located between the electrode 132A and the electrode 132B, and the encapsulating liquid surrounds and covers the lens liquid 132C. The liquid lens 132 further includes an insulating layer 132E to insulate the lens liquid 132C from the electrode 132A.

When the electrode 132A and the electrode 132B are energized, the electric field generated changes the thickness of the lens liquid 132C, thereby changing the focal length of the liquid lens 132, such that ultrasonic waves or far infrared rays are focused on a specific location as required.

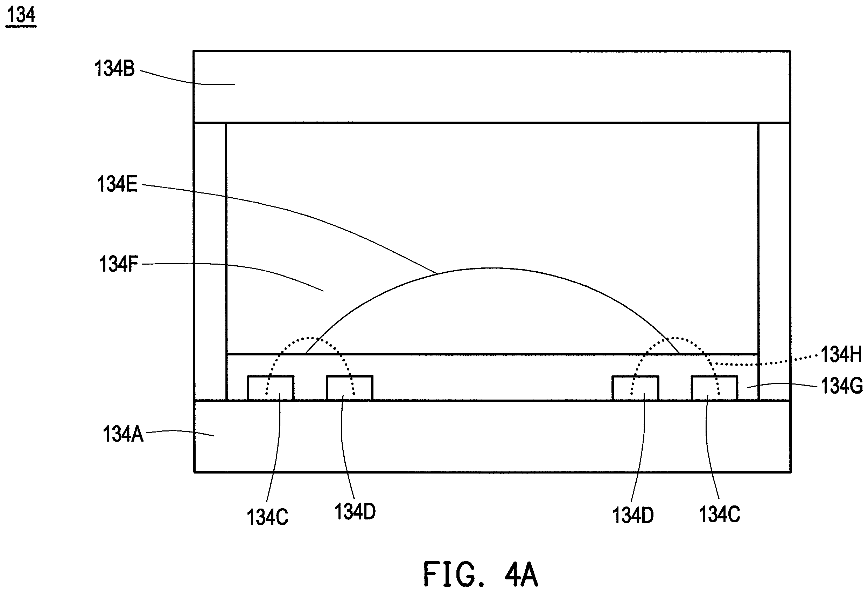

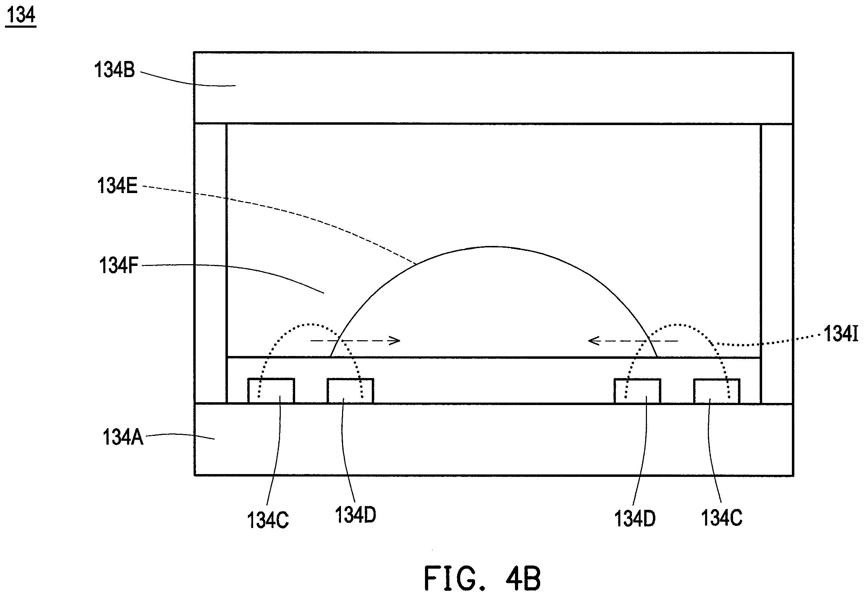

FIG. 4A and FIG. 4B are schematic diagrams of another liquid lens according to some embodiments of the disclosure. As shown in FIG. 4A, the liquid lens 134 is an embodiment of the liquid lens 130A and the electrode 130B in FIG. 1. The liquid lens 134 includes a window 134A and a window 134B. According to some embodiments, the windows 134A and 134B are glass sheets, or other transparent materials that ultrasonic waves or far infrared rays can penetrate, but the disclosure is not limited thereto. The liquid lens 134 further includes a lens liquid 134E and an encapsulating liquid 134F. The lens liquid 134E and the encapsulating liquid 134F are located between the window 134A and the window 134B. The lens liquid 134E and the encapsulating liquid 134F are two immiscible liquids. According to some embodiments, the lens liquid 134E is oil, and the encapsulating liquid 134F is water, but it is not limited thereto. The electrodes 134C and the electrodes 134D are sandwiched between the lens solution 134E/ the encapsulation solution 134F and the window 134A, and are covered with an insulating layer 134G for insulation from the lens solution 134E/ the encapsulation solution 134F.

When the electrodes 134C and the electrodes 134D are energized, the electric field 134H generated (as shown in FIG. 4A) or the electric field 1341 (as shown in FIG. 4B) changes the thickness of the lens liquid 134E, thereby changing the focal length of the liquid lens 134, and the ultrasound waves or far infrared rays are focused on a specific location as required.

FIG. 5 is a time sequence diagram of the voltage of the liquid lens and the voltage of the ultrasonic source according to some embodiments of the disclosure. Since the ultrasonic wave acupuncture device 100 as shown in FIG. 1 uses ultrasonic waves to simulate the effect of acupuncture, the administration of acupuncture is introduced here briefly.

In traditional Chinese medicine, acupuncture is generally administered by puncturing acupoints with needles. A steel needle is vertically inserted through the skin surface on the acupoint, and the needle is inserted, retained, and retracted at the acupoint to stimulate the acupoint. When performing acupuncture, it is called needle insertion to insert the needle from the shallow layer to reach the deeper layer of the acupoint; and it is called needle lifting to remove the needle from the deeper layer of the acupoint by moving it upward. This vertical needle technique is called the lifting and inserting technique. The range and the speed of moving the needle in and out are equal, and the two alternate with even force. The amplitude and frequency of the lifting is determined according to the treatment requirements, but it should not be moved excessively or too rapidly.

When dealing with different illness, it is common to promote the therapeutic effect by combining a variety of needle techniques. Two common needle techniques, called "Setting Fire on the Mountain" and "Coolness through Penetrating Heaven," are described here for examples.

The technique of "Setting Fire on the Mountain" divides an acupoint into three levels, shallow, middle, and deep, depending on the depth of penetration. After the needle obtain the needling sensation, the needle is lifted to the shallow layer for replenishment 3 to 9 times in the shallow layer, then the needle is inserted into the middle layer for replenishment/tonifying method 3 to 9 times, before it is then inserted to the deep layer for another 3 to 9 times of replenishment, and at last, the needle is withdrawn to the shallow level. This process is called a course. The replenishment refers to a needle technique that restores a depressed function to its activity by compensating for the lack of some constituents in the body. The procedure is repeated for several times until there's heat produced beneath the needle, or until a heat sensation emerges the patient's body. It is suitable for the syndrome of pathological deficiency and cold.

The technique of "Coolness through Penetrating Heaven" is to insert the needle into the deep layer after the needle obtains the needle sensation, and perform the dissipate/purging 6 to 8 times in the deep layer, then lift the needle to the middle layer 6 to 8 times, and lift the needle to the shallow layer for another 6 to 8 times, and lastly insert the needle to the deep level. This process is called a course (i.e., a course of treatment). The dissipation refers to a needle technique that restores the hyperactive function to normal by removing the unnecessary or harmful constituents from the body. The operation is repeated several times to produce a cold sensation beneath the needle or to create coolness in the patient's body. It is suitable for the syndrome of pathological heat or excess.

Since acupuncture requires puncturing the skin with stainless steel, possible problems include: strong irritation, pain, skin puncture, and tendency to cross-infection. In addition, the needling risk, technical difficulty, and difficulty in duplication in acupuncture make the repetition and accuracy of acupuncture another problem.

The time sequence diagram of the voltage of the liquid lens and the voltage of the ultrasonic source in FIG. 5 shows a needling effect similar to the technique "Setting Fire on the Mountain" produced by the ultrasonic wave acupuncture device 100. The time sequence 500 is a time sequence diagram of the applied voltage of the liquid lens 130A. The time sequence 510 is a time sequence diagram of the applied voltage of the ultrasonic source 110. According to the applied voltage of the liquid lens 130A, the focal length of the liquid lens 130A may be changed. The higher the applied voltage, the smaller the focal length of the liquid lens 130A, which focuses the ultrasonic waves generated by the ultrasonic source 110 to a shallower location.

As shown in FIG. 5, according to the time sequence 500, the liquid-lens controller 162 outputs the liquid-lens voltage pulses 502, 504, and 506 to the liquid lens 130A at fixed intervals. The pulse 502, the pulse 504, and the pulse 506 have different voltages that change the focal length of the liquid lens 130A. The pulse durations of the pulse 502, the pulse 504, and the pulse 506 are all t1, and the interval (pulse interval) duration between the pulses are all t2. The interval duration t2 between the pulses is greater than the pulse duration t1. In other embodiments, the interval duration t2 between pulses is equal to the pulse duration t1 or less than the pulse duration t1, but the disclosure is not limited thereto. In this embodiment, one "course" is one liquid-lens voltage cycle, including five pulses 502, five pulses 504, and five pulses 506. The pulse 502, the pulse 504, and the pulse 506 respectively have different voltages to change the focal length of the liquid lens 130A in FIG. 1.

On the other hand, the time sequence 510 has a plurality of pulses 512, and the pulse 512 has the same pulse duration t1 and the same interval duration t2 between pulses as the pulse 502, the pulse 504, and the pulse 506. The pulse 512 of the time sequence 510 is synchronized with the pulse 502, the pulse 504, and the pulse 506 of the time sequence 500. That is to say, the pulses of the time sequence 500 of the liquid lens 130A and the pulses of the time sequence 510 of the ultrasonic source 110 are aligned with each other. The voltage of the pulse 512 makes the ultrasonic source 110 in FIG. 1 generate ultrasonic waves of a specific frequency and intensity.

As shown in FIG. 5, the pulse 502 has a voltage VL (a first voltage intensity) and it is repeated five times (a plurality of first liquid-lens voltage pulses). This means that at the time of the emission of the pulse 502, the liquid lens 130A of FIG. 1 has a focal length f1 due to the applied voltage V_{L}. In the time sequence 510, the pulse 512 has a voltage V_{US} and appears synchronously at the time corresponding to the pulse 502. This means that at the time of the emission of the pulse 512, the ultrasonic source 110 generates ultrasonic waves of corresponding intensity due to the applied voltage V_{US}. When the ultrasonic waves generated by the ultrasonic source 110 passes through the liquid lens 130A, it is focused at the focal length f1 (a first focal length), which is equivalent to the location where the needle penetrates the focal length f1. When the pulse 502 and the pulse 512 disappear, it is equivalent to retracting the needle. As shown in FIG. 2A to FIG. 2C, the smaller the applied voltage of the liquid lens, the larger the focal length of the liquid lens, and the larger the applied voltage of the liquid lens, the smaller the focal length of the liquid lens. Therefore, in FIG. 5, emitting the pulse 502 five times is equivalent to "raising or moving the needle to the deep layer and performing the replenishment five times in the deep layer."

Next, in the time sequence 500, the pulse 504 has the voltage V_{M} (a second voltage intensity) and it is repeated five times (a plurality of second liquid-lens voltage pulses). This means that at the time of the emission of the pulse 504, the liquid lens 130A has a focal length f2 (a second focal length) due to the applied voltage V_{M}. As shown in FIG. 2A to FIG. 2C, the smaller the applied voltage of the liquid lens, the larger the focal length of the liquid lens, and the larger the applied voltage of the liquid lens, the smaller the focal length of the liquid lens. Since the applied voltage V_{M} is greater than V_{L}, the focal length f2 is smaller than the focal length f1. In the time sequence 510, the pulse 512 with the voltage V_{US} still appears at the time corresponding to the pulse 504. This means that during the pulse 504, the ultrasonic source 110 generates ultrasonic pulses with corresponding intensity due to the applied voltage V_{US}, and when passing through the liquid lens 130A, they are focused on the focal length f2, which is equivalent to the place where the needle penetrates the focal length f2. Therefore, in FIG. 5, the pulse 504 is performed five times, which is equivalent to "inserting the needle into the middle layer and performing the replenishment five times."

Next, in the time sequence 500, the pulse 506 has the voltage V_{H} (a third voltage intensity), and it is repeated five times (a plurality of third liquid-lens voltage pulses). This means that at the time of the emission of the pulse 506, the liquid lens 130A has a focal length f3 (a third focal length) due to the applied voltage V_{H}. As shown in FIG. 2A to FIG. 2C, the smaller the applied voltage of the liquid lens, the larger the focal length of the liquid lens, and the larger the applied voltage of the liquid lens, the smaller the focal length of the liquid lens. Since the applied voltage V_{H} is greater than V_{M}, the focal length f3 is smaller than the focal length f2. In the time sequence 510, the pulse 512 with the voltage V_{US} still appears at the time corresponding to the pulse 506. This means that at the time of the emission of the pulse 506, the ultrasonic source 110 generates ultrasonic pulses of corresponding intensity due to the applied voltage V_{US}, and when passing through the liquid lens 130A, they are focused on the focal length f3, it is equivalent to the place where the needle penetrates the focal length f3. Therefore, in FIG. 5, the pulse 506 is performed five times, which is equivalent to "re-inserting or moving the needles to the shallow layer and performing the replenishment five times."

After the pulse 506 is performed five times, the time sequence 500 returns to the emission of the pulse 502 again. At this time, it is equivalent to the final insertion of the needle into the deep layer, which is the location of the focal length f1 of the liquid lens 130A. Such a cycle (liquid-lens voltage cycle) includes five pulses 502, five pulses 504, and five pulses 506, which is called one course. According to some embodiments, different liquid lens voltages are included in one course, and the number of times each liquid lens voltage appears may be more than once, depending on requirements, and the disclosure is not limited thereto.

In FIG. 5, it is worth noting that in the time sequence 500 and the time sequence 510, only the voltage of the liquid lens changes, that is, the focal length of the liquid lens 130A changes, while the voltage 512 of the ultrasonic source 110 remains unchanged at all time. Therefore, it is equivalent to that the ultrasonic source 110 continuously generates ultrasonic waves of the same power. By changing the focal length of the liquid lens 130A, the ultrasonic waves may be focused on specific acupoints at different depths.

FIG. 6 is a time sequence diagram of the voltage of the liquid lens and the voltage of the ultrasonic source according to other embodiments of the disclosure. As shown in FIG. 6, the time sequence 600 is a time sequence diagram of the applied voltage of the liquid lens 130A, and the time sequence 610 is a time sequence diagram of the applied voltage of the ultrasonic source 110. As the reference numerals in FIG. 6 and FIG. 5 have similar properties, they are not described in detail again. The differences between FIG. 6 and FIG. 5 are as follows. In the sequence 600, in addition to the voltage V_{L}, the pulse 602 further changes periodically within a perturbation voltage ΔV, that is, the voltage periodically changes between V_{L} and V_{L}+ΔV (a first perturbation voltage range). The perturbation voltage ΔV is smaller or much smaller than the voltage V_{L}. According to other embodiments, the voltage changing range periodically changes between V_{L} and V_{L}-ΔV, or periodically changes between V_{L}-ΔV and V_{L}+ΔV, but the disclosure is not limited thereto. This means that the liquid lens 130A has a focal length f1 due to the applied voltage V_{L} and a focal length f1-Δf (a first perturbation focal length) when the voltage V_{L}+ΔV (a first perturbation voltage) is applied. The perturbation focal length Δf is smaller or much smaller than the focal length f1. In other words, the focal length f1 of the liquid lens 130A changes correspondingly as the voltage changes around V_{L}. In the time sequence 610, the pulse 612 has the voltage V_{US} and appears at the time corresponding to the pulse 602, and each pulse 602 is synchronized with the pulse 612 and they are aligned with each other. This means that at the time of the emission of the pulse 612, the ultrasonic source 110 generates an ultrasonic pulse of corresponding intensity due to the applied voltage V_{US}. When this ultrasonic pulse passes through the liquid lens 130A, they are focused at the focal length f1, which is equivalent to where the needle penetrates the focal length f1, and as the voltage of the pulse 602 changes, they are focused sequentially at f1 and f1-Δf. The combination of the pulse 602 and the pulse 612 is equivalent to needle insertion and withdrawal with small amplitude at f1, that is, introducing perturbation at f1. Similarly, the voltages at pulses 604 and 606 are respectively V_{M}+ΔV (a second perturbation voltage) and V_{H}+ΔV (a third perturbation voltage), respectively changing between V_{M} and V_{M}+ΔV (a second perturbation voltage range) and between V_{H} and V_{H}+ΔV (the third perturbation voltage range), so as to generate disturbances respectively at the focal lengths f2 and f3 corresponding to V_{M} and V_{H}, each generating f2-Δf (a second perturbation focal length) and f3-Δf (a third perturbation focal length) respectively. According to some embodiments, the voltage perturbation ΔV provided by the liquid lens 130A causes the focal length to vary from 0.5 to 10 mm, but it is not limited thereto. In practical applications, the amount of change in focal length depends on the location of the acupoint and the treatment needed.

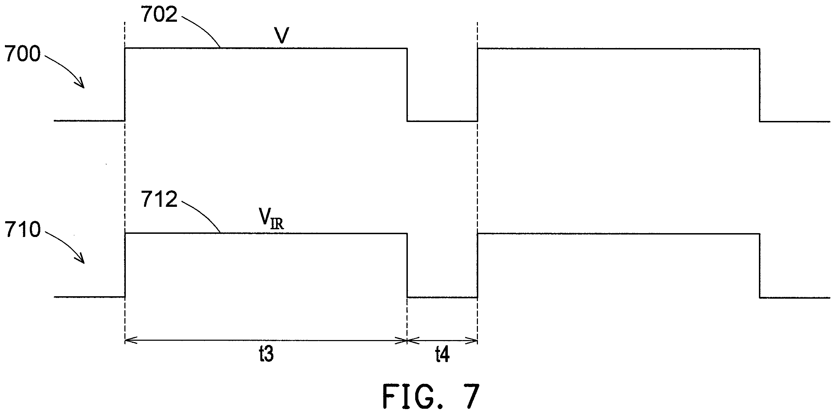

FIG. 7 is a time sequence diagram of the voltage of the liquid lens and the voltage of the infrared source according to other embodiments of the disclosure. The time sequence 700 is a time sequence diagram of the applied voltage of the liquid lens 130A. The time sequence 710 is a time sequence diagram of the applied voltage of the infrared source 120. Compared with the needling mode shown in FIG. 5 and FIG. 6, if the acupuncture mode is selected, that is, when far infrared rays are adopted to irradiate the acupoints, the focal length of the liquid lens 130A is controlled to a fixed value, and the acupoints are irradiated with focused far infrared rays. When the acupuncture mode is selected, the ultrasound source 110 is turned off.

As shown in FIG. 7, in the sequence 700, the liquid-lens controller 162 outputs the liquid-lens voltage pulses 702 to the liquid lens 130A at fixed intervals to change the focal length of the liquid lens 130A. The pulse duration of the pulse 702 is t3, and the interval duration between pulses is t4. The pulse 702 is adapted to change the focal length of the liquid lens 130A in FIG. 1.

On the other hand, the time sequence 710 has a pulse 712. The pulse 712 has the same pulse duration t3and the same pulse interval duration t4 as the pulse 702. The pulse duration t3 is greater than the pulse interval duration t4, and the pulse 712 of the time sequence 710 is synchronized with the pulse 702 of the time sequence 700. In other embodiments, the interval duration t4 between pulses is equal to the pulse duration t3 or is greater than the pulse duration t3, but the disclosure is not limited thereto. The voltage of the pulse 712 is adapted to make the infrared source 120 in FIG. 1 generate far infrared rays with a specific intensity, and the irradiation of the far infrared rays are equivalent to the "moxibustion" in the traditional Chinese medicine that heats and stimulates the acupoints.

In the time sequence 700 of the liquid lens 130A, the pulse 702 has a voltage V, which means that at the time of the emission of the pulse 702, the liquid lens 130A has a focal length f due to the applied voltage V. In the time sequence 710 of the infrared source 120, the pulse 712 has the voltage V_{IR} and appears at the time corresponding to the pulse 702. This means that at the time of the emission of the pulse 712, the infrared source 120 generates infrared light of corresponding intensity due to the applied voltage V_{IR}. When the infrared light generated by the infrared source 120 passes through the liquid lens 130A, they are focused on the focal length f, which is equivalent to heating the place where the focal length f is at. According to some embodiments, the pulse length is 30 seconds, and the interval between the two pulses is 10 seconds, which is equivalent to 30 seconds of irradiation and 10 seconds of rest, but it is not limited thereto.

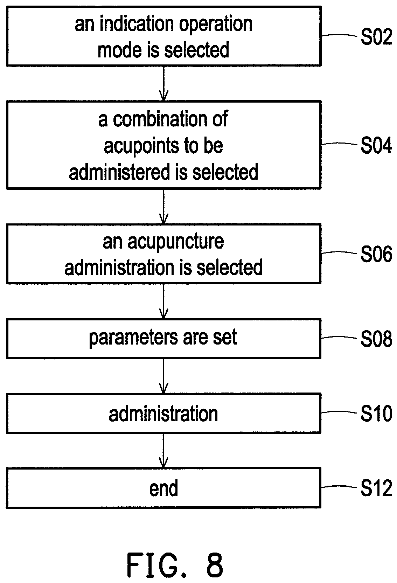

FIG. 8 is an operation flowchart of the ultrasonic wave acupuncture device according to some embodiments of the disclosure. As shown in FIG. 8, in step S02, an indication operation mode is selected to determine the time sequence of the applied voltage of the liquid lens and the ultrasonic source as shown in FIG. 5 and FIG. 6, or determine the time sequence of the applied voltage of the liquid lens and the infrared light source as shown in FIG. 7, so as to determine the focus position of the liquid lens and the intensity of the ultrasonic waves generated by the ultrasonic source. For example, the choice of needle technique is Setting Fire on the Mountain or Coolness through Penetrating Heaven, or the choice of needle technique is to replenish or to dissipate qi, but it is not limited thereto.

In step S04, a combination of acupoints to be administered/executed is selected. For example, Hegu acupoint or a combination of other multiple acupoints is selected, but it is not limited thereto. The focal range of the liquid lens is determined based on the selected combination of acupoints.

In step S06, an acupuncture administration is selected. For example, a "needling" mode or a "moxibustion" mode is selected to determine whether to adopt an ultrasonic source to generate ultrasonic waves or to adopt an infrared source to generate far infrared rays.

In step S08, parameters are set. If the "needling" mode is selected, parameters like pulse length, pulse frequency, speed, and course number need to be set, but it is not limited thereto. If the "moxibustion" mode is selected, parameters like pulse length need to be set, but it is not limited thereto.

In step S10, the administration/execution begins based on the parameters set in steps S02 to S08. At this time, the ultrasonic-source controller 162, the infrared-source controller 164, and the liquid-lens controller 166 send out control signals to respectively control the ultrasonic source 110, the infrared source 120, and the liquid lens 130A, so as to focus the ultrasound waves or far infrared rays generated to a specific location.

In step S12, after the acupuncture administration is completed, the treatment ends.

In summary, the disclosure adopts a liquid lens to focus ultrasound waves or far infrared rays and achieves effects similar to the needle insertion or withdrawal in acupuncture by changing the focal length of the liquid lens to accomplish the effect of a non-invasive treatment.

## Claims

1. An ultrasonic wave acupuncture device (100), comprising:
an ultrasonic source (110), adapted to generate ultrasonic waves;
and
an ultrasonic-source controller (162), electrically connected to the ultrasonic source (110) to generate a plurality of ultrasonic-source voltage pulses to control a frequency, an amplitude, and a pulse length of the ultrasonic waves generated by the ultrasonic source (110); the ultrasonic wave acupuncture device (100) being **characterized in** further comprising: a liquid lens (130A, 132, 134) adapted to focus the ultrasonic wave,
a liquid-lens controller (166), electrically connected to the liquid lens (130A, 132, 134) to generate a plurality of liquid-lens voltage pulses to control a focal length of the liquid lens (130A, 132, 134), so as to focus the ultrasonic waves on a specific location; and
a power supply (170), electrically connected to the ultrasonic-source controller (162) and the liquid-lens controller (166), adapted to provide voltage to the ultrasonic-source controller (162) to control the ultrasonic source (110) and to provide voltage to the liquid-lens controller (166) to control the liquid lens (130A, 132, 134),
wherein the ultrasonic-source voltage pulses generated by the ultrasonic-source controller (162) and the liquid-lens voltage pulses generated by the liquid-lens controller (166) are aligned with each other.

2. The ultrasonic wave acupuncture device (100) according to claim 1, the liquid-lens controller (166) outputs the liquid-lens voltage pulses to the liquid lens (130A, 132, 134) at fixed intervals, and the liquid-lens voltage pulses have different voltages to change the focal length of the liquid lens (130A, 132, 134).

3. The ultrasonic wave acupuncture device (100) according to claim 2, wherein a voltage value of each of the liquid-lens voltage pulses periodically changes within a perturbation voltage range.

4. The ultrasonic wave acupuncture device (100) according to claim 1, wherein the liquid-lens controller (166) is adapted to generate a plurality of liquid-lens voltage cycles; each of the liquid-lens voltage cycles comprises generating a plurality of first liquid-lens voltage pulses with a first voltage intensity at fixed intervals, such that the liquid lens (130A, 132, 134) generates a first focal length corresponding to the first voltage intensity, then generating a plurality of second liquid-lens voltage pulses with a second voltage intensity at the fixed intervals, such that the liquid lens (130A, 132, 134) generates a second focal length corresponding to the second voltage intensity, and then generating a plurality of third liquid-lens voltage pulses with a third voltage intensity at the fixed interval, such that the liquid lens (130A, 132, 134) generates a third focal length corresponding to the third voltage intensity.

5. The ultrasonic wave acupuncture device (100) according to claim 4, wherein the first voltage intensity changes within a first perturbation voltage range, such that the liquid lens (130A, 132, 134) changes within the first focal length and a first perturbation focal length corresponding to the first perturbation voltage; the second voltage intensity changes within a second perturbation voltage range, such that the liquid lens (130A, 132, 134) changes within the second focal length and a second perturbation focal length corresponding to the second perturbation voltage; and the third voltage intensity changes within a third perturbation voltage range, such that the liquid lens (130A, 132, 134) changes within the third focal length and a third perturbation focal length corresponding to the third perturbation voltage.

6. The ultrasonic wave acupuncture device (100) according to claim 1, wherein a flare diameter is less than 0.5 mm after the ultrasonic waves are focused by the liquid lens (130A, 132, 134).

7. The ultrasonic wave acupuncture device (100) according to claim 1, wherein a frequency of the ultrasonic waves is 1 to 3 MHz.

8. The ultrasonic wave acupuncture device (100) according to claim 1, wherein a power of the ultrasonic waves is 100 to 300 mW.

9. The ultrasonic wave acupuncture device (100) according to claim 1, wherein one side (110S) of the ultrasonic source (110) facing the liquid lens (130A, 132, 134) is a flat surface.

10. The ultrasonic wave acupuncture device (100) according to claim 1, wherein the ultrasonic source (110) is a piezoelectric plate.

11. The ultrasonic wave acupuncture device (100) according to claim 10, wherein a material of the piezoelectric plate is piezoelectric ceramic.

12. The ultrasonic wave acupuncture device (100) according to claim 1, wherein the liquid lens (130A, 132, 134) is a plano-convex lens.

13. The ultrasonic wave acupuncture device (100) according to claim 1, wherein a focal length of the liquid lens (130A, 132, 134) changes along a straight line.

14. The ultrasonic wave acupuncture device (100) according to claim 1, further comprising:
an infrared source (120), located on one side of the ultrasonic source (110) facing away from the liquid lens (130A, 132, 134), adapted to generate far infrared rays;
an infrared-source controller (164), electrically connected to the infrared source (120) to generate a plurality of infrared-source voltage pulses to control energy and pulse length of the far infrared rays generated by the infrared source (120);
wherein the ultrasonic source (110) has a hole to allow the far infrared rays to pass through,
wherein the power supply (170) and the infrared-source controller (164) are electrically connected to provide voltage to the infrared-source controller (164) to control the infrared source (120),
wherein the infrared-source voltage pulses of the infrared-source controller (164) and the liquid-lens voltage pulses generated by the liquid-lens controller (166) are aligned with each other.

15. The ultrasonic wave acupuncture device (100) according to claim 14, wherein the liquid-lens controller (166) outputs a plurality of liquid-lens voltage pulses to the liquid lens (130A, 132, 134) at fixed intervals, and a pulse length of each of the liquid-lens voltage pulses is greater than a pulse interval.

16. The ultrasonic wave acupuncture device (100) according to claim 14, wherein the infrared source (120) is a light-emitting diode.

17. The ultrasonic wave acupuncture device (100) according to claim 14, wherein a wavelength range of the far infrared rays is 8 to 14 µm.

18. The ultrasonic wave acupuncture device (100) according to claim 1, further comprising:
an infrared source (120), adapted to generate far infrared rays; and
an infrared-source controller (164), electrically connected to the infrared source (120) to generate a plurality of infrared-source voltage pulses, so as to control energy and pulse length of the far infrared rays generated by the infrared source (120);wherein the ultrasonic source (110) having a hole (112) to allow the far infrared rays to pass through;
wherein the liquid lens (130A, 132, 134), further adapted to focus the far infrared rays;
wherein the liquid-lens controller (166) further controls the focal length of the liquid lens (130A, 132, 134) to focus the ultrasonic waves and the far infrared rays at the specific location; and
wherein the power supply (170), electrically further connected to the infrared-source controller (164), so as to provide voltage to the infrared-source controller (164) to control the infrared source (120),
wherein when the ultrasonic source (110) emits the ultrasonic waves, the infrared source (120) turns off the far infrared rays, and when the infrared source (120) emits the far infrared rays, the ultrasonic source (110) turns off the ultrasonic waves,
wherein the infrared-source voltage pulses of the infrared-source controller (164) and the liquid-lens voltage pulses generated by the liquid-lens controller (166) are aligned with each other.

## Patentansprüche

1. Ultraschallwellen-Akupunkturgerät (100), umfassend:
eine Ultraschallquelle (110), die angepasst ist, Ultraschallwellen zu erzeugen, und
eine Ultraschallquellensteuerung (162), die elektrisch mit der Ultraschallquelle (110) verbunden ist, um eine Vielzahl von Ultraschallquellen-Spannungsimpulsen zu erzeugen, um eine Frequenz, eine Amplitude und eine Impulslänge der von der Ultraschallquelle (110) erzeugten Ultraschallwellen zu steuern, wobei das Ultraschallwellen-Akupunkturgerät (100) **dadurch gekennzeichnet ist, dass** sie ferner umfasst:
eine Flüssigkeitslinse (130A, 132, 134), die zur Fokussierung der Ultraschallwellen angepasst ist,
eine Flüssigkeitslinsensteuerung (166), die elektrisch mit der Flüssigkeitslinse (130A, 132, 134) verbunden ist, um eine Vielzahl von Flüssigkeitslinsen-Spannungsimpulsen zu erzeugen, um eine Brennweite der Flüssigkeitslinse (130A, 132, 134) zu steuern, um die Ultraschallwellen auf eine bestimmte Stelle zu fokussieren; und
eine Stromversorgung (170), die elektrisch mit der Ultraschallquellensteuerung (162) und der Flüssigkeitslinsensteuerung (166) verbunden ist und so angepasst ist, dass sie der Ultraschallquellensteuerung (162) Spannung zuführt, um die Ultraschallquelle (110) zu steuern, und der Flüssigkeitslinsensteuerung (166) Spannung zuführt, um die Flüssigkeitslinse (130A, 132, 134) zu steuern,
wobei die von der Ultraschallquellensteuerung (162) erzeugten Ultraschallquellen-Spannungsimpulse und die von der Flüssigkeitslinsensteuerung (166) erzeugten Flüssigkeitslinsen-Spannungsimpulse aufeinander abgestimmt sind.

2. Ultraschallwellen-Akupunkturgerät (100) gemäß Anspruch 1, wobei die Flüssigkeitslinsensteuerung (166) die Flüssigkeitslinsen-Spannungsimpulse in festen Intervallen an die Flüssigkeitslinse (130A, 132, 134) ausgibt und die Flüssigkeitslinsen-Spannungsimpulse unterschiedliche Spannungen haben, um die Brennweite der Flüssigkeitslinse (130A, 132, 134) zu ändern.

3. Ultraschallwellen-Akupunkturgerät (100) gemäß Anspruch 2, wobei sich ein Spannungswert jedes der Flüssigkeitslinsen-Spannungsimpulse periodisch innerhalb eines Störungsspannungsbereichs ändert.

4. Ultraschallwellen-Akupunkturgerät (100) gemäß Anspruch 1, wobei die Flüssigkeitslinsensteuerung (166) so angepasst ist, dass sie eine Vielzahl von Flüssigkeitslinsenspannungszyklen erzeugt, jeder der Flüssigkeitslinsenspannungszyklen das Erzeugen einer Vielzahl von ersten Flüssigkeitslinsen-Spannungsimpulsen mit einer ersten Spannungsintensität in festen Intervallen umfasst, so dass die Flüssigkeitslinse (130A, 132, 134) eine erste Brennweite erzeugt, entsprechend der ersten Spannungsintensität, und dann das Erzeugen einer Vielzahl von zweiten Flüssigkeitslinsen-Spannungsimpulsen mit einer zweiten Spannungsintensität in den festen Intervallen, so dass die Flüssigkeitslinse (130A, 132, 134) eine zweite Brennweite erzeugt, entsprechend der zweiten Spannungsintensität, und dann das Erzeugen einer Vielzahl von dritten Flüssigkeitslinsen-Spannungsimpulsen mit einer dritten Spannungsintensität in dem festen Intervall umfasst, so dass die Flüssigkeitslinse (130A, 132, 134) eine dritte Brennweite erzeugt, entsprechend der dritten Spannungsintensität.

5. Ultraschallwellen-Akupunkturgerät (100) gemäß Anspruch 4, wobei sich die erste Spannungsintensität innerhalb eines ersten Störspannungsbereichs ändert, so dass sich die Flüssigkeitslinse (130A, 132, 134) innerhalb der ersten Brennweite und einer ersten Störbrennweite entsprechend der ersten Störspannung ändert, die zweite Spannungsintensität sich innerhalb eines zweiten Störspannungsbereichs ändert, so dass sich die Flüssigkeitslinse (130A, 132, 134) innerhalb der zweiten Brennweite und einer zweiten Störbrennweite entsprechend der zweiten Störspannung ändert, und sich die dritte Spannungsintensität innerhalb eines dritten Störspannungsbereichs ändert, so dass sich die Flüssigkeitslinse (130A, 132, 134) innerhalb der dritten Brennweite und einer dritten Störbrennweite entsprechend der dritten Störspannung ändert.

6. Ultraschallwellen-Akupunkturgerät (100) gemäß Anspruch 1, wobei der Durchmesser des Flare weniger als 0,5 mm beträgt, nachdem die Ultraschallwellen durch die Flüssigkeitslinse (130A, 132, 134) fokussiert wurden.

7. Ultraschallwellen-Akupunkturgerät (100) gemäß Anspruch 1, wobei eine Frequenz der Ultraschallwellen 1 bis 3 MHz beträgt.

8. Ultraschallwellen-Akupunkturgerät (100) gemäß Anspruch 1, wobei die Leistung der Ultraschallwellen 100 bis 300 mW beträgt.

9. Ultraschallwellen-Akupunkturgerät (100) gemäß Anspruch 1, wobei eine Seite (1105) der Ultraschallquelle (110), die der Flüssigkeitslinse (130A, 132, 134) zugewandt ist, eine flache Oberfläche ist.

10. Ultraschallwellen-Akupunkturgerät (100) gemäß Anspruch 1, wobei die Ultraschallquelle (110) eine piezoelektrische Platte ist.

11. Ultraschallwellen-Akupunkturgerät (100) gemäß Anspruch 10, wobei ein Material der piezoelektrischen Platte piezoelektrische Keramik ist.

12. Ultraschallwellen-Akupunkturgerät (100) gemäß Anspruch 1, wobei die Flüssigkeitslinse (130A, 132, 134) eine plan-konvexe Linse ist.

13. Ultraschallwellen-Akupunkturgerät (100) gemäß Anspruch 1, wobei sich eine Brennweite der Flüssigkeitslinse (130A, 132, 134) entlang einer geraden Linie ändert.

14. Ultraschallwellen-Akupunkturgerät (100) gemäß Anspruch 1, ferner umfassend:
eine Infrarotquelle (120), die sich auf einer von der Flüssigkeitslinse (130A, 132, 134) abgewandten Seite der Ultraschallquelle (110) befindet und angepasst ist, Ferninfrarotstrahlen zu erzeugen,
eine Infrarotquellensteuerung (164), die elektrisch mit der Infrarotquelle (120) verbunden ist, um eine Vielzahl von Infrarotquellen-Spannungsimpulsen zu erzeugen, um die Energie und die Impulslänge der von der Infrarotquelle (120) erzeugten Ferninfrarotstrahlen zu steuern,
wobei die Ultraschallquelle (110) ein Loch aufweist, durch das die Ferninfrarotstrahlen hindurchtreten können,
wobei die Stromversorgung (170) und die Infrarotquellensteuerung (164) elektrisch verbunden sind, um die Infrarotquellensteuerung (164) mit Spannung zu versorgen, um die Infrarotquelle (120) zu steuern,
wobei die Infrarotquellen-Spannungsimpulse der Infrarotquellensteuerung (164) und die von der Flüssigkeitslinsensteuerung (166) erzeugten Flüssigkeitslinsen-Spannungsimpulse aufeinander abgestimmt sind.

15. Ultraschallwellen-Akupunkturgerät (100) gemäß Anspruch 14, wobei die Flüssigkeitslinsensteuerung (166) eine Vielzahl von Flüssigkeitslinsen-Spannungsimpulsen an die Flüssigkeitslinse (130A, 132, 134) in festen Intervallen ausgibt und eine Impulslänge jedes der Flüssigkeitslinsen-Spannungsimpulse größer als ein Impulsintervall ist.

16. Ultraschallwellen-Akupunkturgerät (100) gemäß Anspruch 14, wobei die Infrarotquelle (120) eine Leuchtdiode ist.

17. Ultraschallwellen-Akupunkturgerät (100) gemäß Anspruch 14, wobei der Wellenlängenbereich der Ferninfrarotstrahlen 8 bis 14 µm beträgt.

18. Ultraschallwellen-Akupunkturgerät (100) gemäß Anspruch 1, ferner umfassend:
eine Infrarotquelle (120), die angepasst ist, Ferninfrarotstrahlen zu erzeugen, und
eine Infrarotquellensteuerung (164), die elektrisch mit der Infrarotquelle (120) verbunden ist, um eine Vielzahl von Infrarotquellen-Spannungsimpulsen zu erzeugen, um die Energie und die Impulslänge der von der Infrarotquelle (120) erzeugten Ferninfrarotstrahlen zu steuern, wobei die Ultraschallquelle (110) ein Loch (112) aufweist, um die Ferninfrarotstrahlen hindurchzulassen;
wobei die Flüssigkeitslinse (130A, 132, 134) ferner angepasst ist, die Ferninfrarotstrahlen zu fokussieren;
wobei die Flüssigkeitslinsensteuerung (166) ferner die Brennweite der Flüssigkeitslinse (130A, 132, 134) steuert, um die Ultraschallwellen und die Ferninfrarotstrahlen an der spezifischen Stelle zu fokussieren; und
wobei die Stromversorgung (170) elektrisch weiter mit der Infrarotquellensteuerung (164) verbunden ist, um die Infrarotquellensteuerung (164) mit Spannung zu versorgen, um die Infrarotquelle (120) zu steuern,
wobei, wenn die Ultraschallquelle (110) die Ultraschallwellen aussendet, die Infrarotquelle (120) die Ferninfrarotstrahlen ausschaltet, und wenn die Infrarotquelle (120) die Ferninfrarotstrahlen aussendet, die Ultraschallquelle (110) die Ultraschallwellen ausschaltet,
wobei die Infrarotquellen-Spannungsimpulse der Infrarotquellensteuerung (164) und die von der Flüssigkeitslinsensteuerung (166) erzeugten Flüssigkeitslinsen-Spannungsimpulse aufeinander abgestimmt sind.

## Revendications

1. Un dispositif (100) d'acupuncture par ondes ultrasonores, comprenant :
une source d'ultrasons (110), adaptée pour générer des ondes ultrasonores ; et
un dispositif (162) de commande de source d'ultrasons, connecté électriquement à la source d'ultrasons (110) pour générer une pluralité d'impulsions de tension de source d'ultrasons pour commander une fréquence, une amplitude et une longueur d'impulsion des ondes ultrasonores générées par la source d'ultrasons (110) ; le dispositif (100) d'acupuncture par ondes ultrasonores étant **caractérisé en ce qu'**il comprend en outre :
une lentille liquide (130A, 132, 134) adaptée pour focaliser l'onde ultrasonore,
un dispositif (166) de commande de lentille liquide, connecté électriquement à la lentille liquide (130A, 132, 134) pour générer une pluralité d'impulsions de tension de lentille liquide pour commander une distance focale de la lentille liquide (130A, 132, 134), de manière à concentrer les ondes ultrasonores sur un endroit précis ; et
une alimentation électrique (170), connectée électriquement au dispositif (162) de commande de source d'ultrasons et au dispositif (166) de commande de lentille liquide, adaptée pour fournir une tension au dispositif (162) de commande de source d'ultrasons afin de commander la source d'ultrasons (110) et fournir une tension au dispositif (166) de commande de lentille liquide afin de commander la lentille liquide (130A, 132, 134),
les impulsions de tension de source d'ultrasons générées par le dispositif (162) de commande de source d'ultrasons et les impulsions de tension de lentille liquide générées par le dispositif (166) de commande de lentille liquide étant alignées les unes avec les autres.

2. Le dispositif (100) d'acupuncture par ondes ultrasonores selon la revendication 1, le dispositif (166) de commande de lentille liquide délivrant les impulsions de tension de lentille liquide à la lentille liquide (130A, 132, 134) à intervalles fixes, et les impulsions de tension de lentille liquide ont des tensions différentes pour modifier la distance focale de la lentille liquide (130A, 132, 134).

3. Le dispositif (100) d'acupuncture par ondes ultrasonores selon la revendication 2, dans lequel une valeur de tension de chacune des impulsions de tension de lentille liquide change périodiquement dans une gamme de tension de perturbation.

4. Le dispositif (100) d'acupuncture par ondes ultrasonores selon la revendication 1, dans lequel le dispositif (166) de commande de lentille liquide est adapté pour générer une pluralité de cycles de tension de lentille liquide ; chacun des cycles de tension de lentille liquide comprend le fait de générer une pluralité de premières impulsions de tension de lentille liquide avec une première intensité de tension à intervalles fixes, de sorte que la lentille liquide (130A, 132, 134) génère une première distance focale correspondant à la première intensité de tension, puis génère une pluralité de deuxièmes impulsions de tension de lentille liquide avec une deuxième intensité de tension à intervalles fixes, de telle sorte que la lentille liquide (130A, 132, 134) génère une deuxième distance focale correspondant à la deuxième intensité de tension, puis génère une pluralité de troisièmes impulsions de tension de lentille liquide avec une troisième intensité de tension à l'intervalle fixe, de telle sorte que la lentille liquide (130A, 132, 134) génère une troisième distance focale correspondant à la troisième intensité de tension.

5. Le dispositif (100) d'acupuncture par ondes ultrasonores selon la revendication 4, dans lequel la première intensité de tension change dans une première plage de tension de perturbation, de telle sorte que la lentille liquide (130A, 132, 134) change dans la première distance focale et une première distance focale de perturbation correspondant à la première tension de perturbation ; la deuxième intensité de tension change dans une deuxième plage de tensions de perturbation, de telle sorte que la lentille liquide (130A, 132, 134) change dans la deuxième distance focale et une deuxième distance focale de perturbation correspondant à la deuxième tension de perturbation ; et la troisième intensité de tension change dans une troisième plage de tensions de perturbation, de telle sorte que la lentille liquide (130A, 132, 134) change dans la troisième distance focale et une troisième distance focale de perturbation correspondant à la troisième tension de perturbation.

6. Le dispositif (100) d'acupuncture par ondes ultrasonores selon la revendication 1, dans lequel un diamètre d'évasement est inférieur à 0,5 mm après que les ondes ultrasonores aient été focalisées par la lentille liquide (130A, 132, 134).

7. Le dispositif (100) d'acupuncture par ondes ultrasonores selon la revendication 1, dans lequel la fréquence des ondes ultrasonores est de 1 à 3 MHz.

8. Le dispositif (100) d'acupuncture par ondes ultrasonores selon la revendication 1, dans lequel la puissance des ondes ultrasonores est de 100 à 300 mW.

9. Le dispositif (100) d'acupuncture par ondes ultrasonores selon la revendication 1, dans lequel un côté (110S) de la source d'ultrasons (110) tourné vers la lentille liquide (130A, 132, 134) est une surface plane.

10. Le dispositif (100) d'acupuncture par ondes ultrasonores selon la revendication 1, dans lequel la source d'ultrasons (110) est une plaque piézoélectrique.

11. Le dispositif (100) d'acupuncture par ondes ultrasonores selon la revendication 10, dans lequel un matériau de la plaque piézoélectrique est une céramique piézoélectrique.

12. Le dispositif (100) d'acupuncture par ondes ultrasonores selon la revendication 1, dans lequel la lentille liquide (130A, 132, 134) est une lentille plan-convexe.

13. Le dispositif (100) d'acupuncture par ondes ultrasonores selon la revendication 1, dans lequel une distance focale de la lentille liquide (130A, 132, 134) change le long d'une ligne droite.

14. Le dispositif (100) d'acupuncture par ondes ultrasonores selon la revendication 1, comprenant en outre :
une source infrarouge (120), située d'un côté de la source d'ultrasons (110) tourné à l'opposé de la lentille liquide (130A, 132, 134), adaptée pour générer des rayons infrarouges lointains ;
un dispositif (164) de commande de source infrarouge, connecté électriquement à la source infrarouge (120) pour générer une pluralité d'impulsions de tension de source infrarouge afin de commander l'énergie et la longueur d'impulsion des rayons infrarouges lointains générés par la source infrarouge (120) ;
la source d'ultrasons (110) présente un trou pour laisser passer les rayons infrarouges lointains,
l'alimentation électrique (170) et le dispositif (164) de commande de source infrarouge étant connectés électriquement pour fournir une tension au dispositif (164) de commande de source infrarouge afin de commander la source infrarouge (120),
les impulsions de tension de source infrarouge du dispositif (164) de commande de source infrarouge et les impulsions de tension de lentille liquide générées par le dispositif (166) de commande de lentille liquide étant alignées les unes avec les autres.

15. Le dispositif (100) d'acupuncture par ondes ultrasonores selon la revendication 14, dans lequel le dispositif (166) de commande de lentille liquide délivre une pluralité d'impulsions de tension de lentille liquide à la lentille liquide (130A, 132, 134) à intervalles fixes, et une longueur d'impulsion de chacune des impulsions de tension de lentille liquide est supérieure à un intervalle d'impulsion.

16. Le dispositif (100) d'acupuncture par ondes ultrasonores selon la revendication 14, dans lequel la source infrarouge (120) est une diode électroluminescente.

17. Le dispositif (100) d'acupuncture par ondes ultrasonores selon la revendication 14, dans lequel une plage de longueurs d'onde des rayons infrarouges lointains va de 8 à 14 µm.

18. Le dispositif (100) d'acupuncture par ondes ultrasonores selon la revendication 1, comprenant en outre :
une source infrarouge (120), adaptée pour générer des rayons infrarouges lointains ; et
un dispositif (164) de commande de source infrarouge, connecté électriquement à la source infrarouge (120) pour générer une pluralité d'impulsions de tension de source infrarouge, de manière à commander l'énergie et la longueur d'impulsion des rayons infrarouges lointains générés par la source infrarouge (120) ; la source d'ultrasons (110) ayant un trou (112) pour permettre aux rayons infrarouges lointains de passer à travers ;
la lentille liquide (130A, 132, 134), adaptée en outre pour focaliser les rayons infrarouges lointains ;
le dispositif (166) de commande de lentille liquide commande en outre la distance focale de la lentille liquide (130A, 132, 134) pour focaliser les ondes ultrasonores et les rayons infrarouges lointains à l'emplacement spécifique ; et
l'alimentation électrique (170), connectée électriquement en outre au dispositif (164) de commande de source infrarouge, de manière à fournir une tension au dispositif (164) de commande de source infrarouge pour commander la source infrarouge (120) ;
lorsque la source d'ultrasons (110) émet les ondes ultrasonores, la source infrarouge (120) éteint les rayons infrarouges lointains, et lorsque la source infrarouge (120) émet les rayons infrarouges lointains, la source d'ultrasons (110) éteint les ondes ultrasonores,
les impulsions de tension de source infrarouge du dispositif (164) de commande de source infrarouge et les impulsions de tension de lentille liquide générées par le dispositif (166) de commande de lentille liquide étant alignées les unes avec les autres.
